Europäisches Patentamt

European Patent Office

Office européen des brevets

(19)

(11) Publication number: **0 003 066**
**A2**

(12)

# EUROPEAN PATENT APPLICATION

(21) Application number: 78200383.4

(22) Date of filing: 20.12.78

(51) Int. Cl.²: **C 07 C 47/52**
**C 07 C 45/00, C 07 C 43/28**
**// C07C69/74**

(30) Priority: 29.12.77 GB 5414877

(43) Date of publication of application:
25.07.79 Bulletin 79/15

(84) Designated contracting states:
BE CH DE FR GB IT NL

(71) Applicant: SHELL INTERNATIONALE RESEARCH
MAATSCHAPPIJ B.V.
Carel van Bylandtlaan 30
NL-2596 HR DEN HAAG(NL)

(72) Inventor: Grotenhuis, Paulus Alexander Maria
Badhuisweg 3
NL-1031 CM Amsterdam(NL)

(72) Inventor: Menninga, Lubbertus
Badhuisweg 3
NL-1031 CM Amsterdam(NL)

(74) Representative: Keuzenkamp, Abraham et al,
P.O. Box 302
NL-2501 CH Den Haag(NL)

(54) Process for the preparation of meta-aryloxybenzaldehydes, and meta- aryloxybenzaldehydes produced by the process.

(57) Process for the preparation of meta-aryloxybenzaldehydes, especially meta-phenoxybenzaldehyde, which comprises reacting a mixture of meta-aryloxybenzyl and meta-aryloxybenzal halides with hexamethylenetetramine and hydrolysing the resulting product in the presence of a non-acidic solvent. Meta-phenoxy benzaldehyde is an important intermediate in the manufacture of a group of insecticidally-active compounds known as pyrethroids.

EP 0 003 066 A2

Croydon Printing Company Ltd.

1

## PROCESS FOR THE PREPARATION OF META-ARYLOXYBENZALDEHYDES, AND META-ARYLOXYBENZALDEHYDES PRODUCED BY THE PROCESS

The invention relates to a process for the preparation of meta-aryloxybenzaldehydes, which compounds are valuable intermediates, for example, in the preparation of pesticides containing a meta-aryloxybenzyl group. Such pesticides include meta-aryloxybenzyl esters of substituted cyclopropanecarboxylic acids, which are the so-called pyrethroid insecticides having outstanding insecticidal properties (see British patent specification 1,413,491).

A known process for the preparation of meta-aryloxybenzaldehydes is described in Netherlands patent application 7701128, and comprises two steps, the first step consisting in the reaction of a mixture of meta-aryloxybenzyl halides and meta-aryloxybenzal halides with hexamethylenetetramine, and the second step consisting in hydrolysis of the resulting product in an acidic medium to form meta-aryloxybenzaldehyde.

The acid used in the second step may be, for example, acetic acid, phosphoric acid, hydrochloric acid or sulphuric acid and, consequently, the vessel in which this known process is to be carried out must be resistant to these acids.

It has now been found that the process can be carried out in the absence of such an acid.

Accordingly, the present invention provides a process for the preparation of meta-aryloxybenzaldehydes, which comprises in a first step reacting a mixture of the corresponding meta-aryloxybenzyl halides and meta-aryloxybenzal halides with hexamethylenetetramine, and, in a second step, hydrolysing the resulting product in the presence of a non-acidic solvent for the said product.

As the process according to the invention does not require acidic media, the vessel in which the process is carried out need not be acid-resistant. Moreover, the second step can be carried out in a shorter time to obtain an even slightly higher yield of meta-aryloxybenzaldehyde.

Excellent yields of aldehyde are usually obtained when the non-acidic solvent in the second step of the process according to the invention is a mixture of water and an alkanol with up to four carbon atoms per molecule, particularly ethanol. Examples of other suitable alkanols are methanol, propanol, 2-propanol, butanol, 2-butanol and 2-methylpropanol.

The product of the first step of the process according to the invention is a mixture comprising a quaternary meta-aryloxybenzylammonium salt and substantially unconverted meta-aryloxybenzal halide; both these compounds may be isolated and then subjected to the hydrolysis in the second step of the process according to the invention but this is unnecessary because hydrolysis of the reaction mixture from the first step proceeds smoothly and efficiently when carried out in the presence of a non-acidic solvent for the product formed in the first step; good dissolution of the meta-aryloxybenzal halide and the quaternary ammonium salt can be attained by using an alkanol/ water mixture containing 40%v to 80%v, and preferably 60%v to 70%v, of an alkanol with up to four carbon atoms per molecule.

Instead of hexamethylenetetramine, ammonia and formaldehyde may be employed in the first step of the process according to the invention and it will be clear that the results will be similar because of the chemical equilibrium between hexamethylenetetramine and ammonia and formaldehyde. Thus, ammonia and formaldehyde may be regarded as precursors to hexamethylenetetramine.

Although the first step in the process according to the invention may be effected in a non-aqueous solvent, such as chloroform, it is conveniently carried out in an aqueous medium in view of the subsequent hydrolysis reaction in the second step of the process. The ammonia and formaldehyde or the hexamethylenetetramine may therefore be used in the form of aqueous solutions. Very high yields of meta-aryloxybenzaldehydes are attained when the first step is carried out in the presence of alkanol-free water over a period of, for example, 2.5-3.5 hours. In the presence of an alkanol with up to four carbon atoms per molecule, very good yields are obtained at shorter reaction times, for example between 0.5 and 2.5 hours. The first step of the process is exothermic and, generally, no heat is required to initiate the reaction; reaction temperatures in the range of from 10 to 150°C may conveniently be used in practice.

Between the first and the second step the content of alkanol with up to four carbon atoms per molecule may be adjusted to a value in the range of from, for example 40%v to 80%v by adding alkanol when the first step has been carried out in the presence of alkanol-free water or by adding water when the first step has been carried out in the presence of water-containing alkanol. It is, however, also possible to carry out the first step in the presence of a water-alkanol mixture of such composition (for example between 60%v and 70%v alkanol) that neither water nor alkanol need be added between the first and the second step.

Hydrolysis in the second step of the process according to the invention may be effected at temperatures in the range of from, for example, 40 to 200°C. Temperatures in the range of

4

from 50 to 120°C are preferred. Short hydrolysis times, between for example 2 and 8 hours, are sufficient when the hydrolysis is carried out at a temperature between, for example 100°C and 200°C; longer hydrolysis times, between for example 8 and 25 hours, are required at temperatures between for example, 40°C and 100°C. The hydrolysis can be carried out under autogenous superatmospheric pressure.

Excellent results in terms of aldehyde yields have been obtained with mixtures of meta-aryloxybenzyl chlorides and meta-aryloxybenzal chlorides; the corresponding bromides are also very suitable.

The mixture of meta-aryloxybenzyl halides and meta-aryloxy-benzal halides can be obtained by any convenient means but it has been found that such a mixture can be readily prepared by halogenation of the appropriate meta-aryloxytoluene. According to an aspect of the present invention, therefore, the mixture of meta-aryloxybenzyl halide and meta-aryloxybenzal halide used as starting material in the process according to the invention can be prepared by a process which comprises halogenating the corresponding meta-aryloxytoluene with gaseous halogen at an elevated temperature in the presence of a free-radical initiator. The temperature of the halogenation reaction depends largely on the nature of the halogen employed and the need to avoid ring halogenation of the meta-aryloxytoluene. A general temperature range for the halogenation reaction is 50 to 250°C.

So far as chlorination of the meta-aryloxytoluene is concerned, better results are obtained by contacting the meta-aryloxytoluene in a non-polar solvent at a temperature in the range 40-100°C with gaseous chlorine, the free-radical initiator preferably being a peroxide or azo initiator such as benzoyl peroxide or azo-isobutyronitrile (AIBN). The non-polar solvent selected for this chlorination reaction must be such that it does not promote the formation of ring-chlorinated products and is itself substantially unaffected by the prevailing chlorination conditions. Generally speaking, halogenated hydrocarbons such as carbon tetrachloride and chlorobenzene are satisfactory solvents for this reaction; excellent results have been obtained with carbon tetrachloride as the solvent. Benzene is also a suitable solvent. In order to favour the side-chain chlorination of the toluene and suppress ring chlorination it has been found desirable to prevent the chlorination of the meta-aryloxytoluene, which occurs at high concentrations, for example over 60% by weight of meta-aryloxytoluene in solvent; concentrations of up to 50% by weight have been found to be generally preferable. Further, the conversion of the meta-aryloxytoluene should not be allowed to proceed to completion because this tends to produce unwanted chlorinated products; thus the reaction should be stopped at a conversion in the range 95-99% based on meta-aryloxytoluene, suitably 98 or 99%. Overall yields of the meta-aryloxybenzyl chloride and meta-aryloxybenzal chloride are generally over 90% and often over 95%. The bromination of the meta-aryloxytoluene may be carried out as described in Netherlands patent application 7701128.

The nature of the meta-aryloxy substituent in the starting material is unimportant but the commercially useful product in terms of its importance to the synthesis of pesticidal pyrethroids is meta-phenoxybenzaldehyde. It follows, therefore, that the preferred starting material is a mixture of meta-phenoxybenzyl halide and meta-phenoxybenzal halide.

It will be appreciated, therefore, that the present invention provides a valuable route to meta-phenoxybenzaldehyde starting from meta-phenoxytoluene without the need to isolate a particular intermediate chloride or bromide for conversion into the aldehyde. An important attribute of the process is its flexibility in being able to convert a mixture in any proportions of meta-phenoxybenzyl halide and metaphenoxybenzal halide (i.e., a mixture of the mono- and dihalides) to the desired aldehyde.

Accordingly, a particular aspect of the invention concerns a process for the preparation of meta-phenoxybenzaldehyde which comprises:

(a) preparing a mixture of meta-phenoxybenzyl halide and meta-phenoxybenzal halide by halogenating meta-phenoxytoluene with gaseous halogen at an elevated temperature in the presence of a free-radical initiator;

(b) reacting the mixture of halides prepared in (a) with hexamethylenetetramine; and

(c) hydrolyzing the reaction product from (b) in the presence of non-acidic solvent for the said product.

The process according to the invention is further illustrated in the following Examples. The conversions of meta-phenoxybenzyl chloride and meta-phenoxybenzal chloride and the yields of meta-phenoxybenzaldehyde were determined by means of gas-liquid chromatography. In each of the Examples the conversions of meta-phenoxybenzyl chloride and meta-phenoxybenzal chloride were 100% and 99-100%, respectively. The yields of meta-phenoxybenzaldehyde were calculated on starting meta-phenoxybenzyl chloride and meta-phenoxybenzal chloride.

EXAMPLE I

(a) Preparation of meta-phenoxybenzyl and meta-phenoxybenzal chloride

A stream of chlorine (1.1 mol. $Cl_2$, 39 g) was passed for 4 hours through a solution of meta-phenoxytoluene (0.70 mol.), and azo-isobutyronitrile (5%m on meta-phenoxytoluene) in carbon tetrachloride (700 ml). At the end of this period 99% of the meta-phenoxytoluene had been converted. Evaporation of the solvent left a residue containing meta-phenoxybenzyl chloride (yield 48.1%) and meta-phenoxybenzal chloride (yield 47.6%).

(b) Preparation of meta-phenoxybenzaldehyde

A glass autoclave was charged with a solution of hexamethylenetetramine (0.27 mol.) in a 65%v/35%v ethanol/water mixture (230 ml) and then, over a period of one hour at 25°C and with stirring, with the residue obtained in section (a) containing 0.11 mol. of meta-phenoxybenzyl chloride and 0.10 mol. of meta-phenoxybenzal chloride. After stirring for another hour at $25^{\circ}$C, water (16 ml) was added and the mixture heated at $110^{\circ}$C for 4 hours under an autogenous pressure of 5 bar.

The meta-phenoxybenzaldehyde was isolated by the following procedure. After cooling to $20^{\circ}$C the ethanol was distilled off (as a 80%v/20%v ethanol/water mixture), 12.5%w hydrochloric acid (32 ml) was added to the residue and the mixture was kept under reflux ($103^{\circ}$C) for one hour. This stage in the isolation procedure is an "acid-wash" to remove any bases present in the residue and the necessary acid-resistant equipment is on a much smaller scale than that required for the process steps in the prior art.

After cooling to $20^{\circ}$C the mixture was allowed to separate into an aqueous and an organic phase; the aqueous phase was extracted with toluene (80 ml), the organic phase was combined with the extract phase, the organic liquid thus obtained was washed with water (40 ml) and the solvent evaporated at a final pressure of 10 mbar to give meta-phenoxybenzaldehyde in a yield of 82%.

EXAMPLE II

A flask was charged with a solution of hexamethylenetetramine (0.35 mol.) in a 67%v/33%v ethanol/water mixture (260 ml) and then, over a period of one hour at $60^{\circ}$C and with stirring, with the residue obtained in section (a) of Example I containing 0.14 mol. of meta-phenoxybenzyl chloride and 0.08 mol. of meta-phenoxybenzal chloride. After stirring for another hour at $60^{\circ}$C, water (16 ml) was added and the mixture kept under reflux for 18 hours ($80^{\circ}$C).

Then the ethanol was distilled off (as a 80%v/20%v ethanol/water mixture) and the resulting residue was divided into two equal portions, one portion being worked up by the procedure in Example I, the other portion being worked up by the same procedure except that the "acid wash" was omitted. Details of the two work-up procedures are given below.

To the first portion 12.5%w hydrochloric acid (16 ml) was added and the isolation of the meta-phenoxybenzaldehyde was carried out as described in Example I, section (b). The meta-phenoxybenzaldehyde was obtained in a yield of 85%.

The second portion was allowed to separate into an aqueous and an organic phase and the meta-phenoxybenzaldehyde was isolated as described in Example I, section (b). The meta-phenoxybenzaldehyde was obtained in a yield of 82%.

EXAMPLE III

A flask was charged with a solution of hexamethylenetetramine (0.27 mol.) in a 65%v/35%v ethanol/water mixture (230 ml) and then, over a period of one hour at 25°C and with stirring, with a mixture of meta-phenoxybenzyl chloride (0.11 mol.) and meta-phenoxybenzal chloride (0.10 mol.) as obtained in Example I, section (a). After stirring for another hour at 25°C, water (16 ml) was added and the mixture kept under reflux (80°C) for 19 hours.

The meta-phenoxybenzaldehyde was isolated as described in Example I, section (b), and obtained in a yield of 89%.

EXAMPLE IV

A flask was charged with a solution of hexamethylenetetramine (0.342 mol.) in a 67%v/33%v ethanol/water mixture (260 ml) and then, with stirring, with a mixture of meta-phenoxybenzyl chloride (0,14 mol.) and meta-phenoxybenzal chloride (0.08 mol.) as obtained in Example I, section (a). After stirring for two hours at 60°C, water (16 ml) was added and the mixture kept under reflux (80°C) for 18 hours.

The meta-phenoxybenzaldehyde was isolated as described in Example I, section (b) and obtained in a yield of 87%.

EXAMPLE V

A glass autoclave was charged with a solution of hexamethylenetetramine (0.27 mol.) in water (82 ml) and then, at once and with stirring, with a mixture of meta-phenoxybenzyl chloride (0.11 mol.) and meta-phenoxybenzal chloride (0.11 mol.) as obtained in Example I, section (a). After stirring for three hours at 90°C, ethanol (154 ml) was added and the mixture heated at 110°C for 4 hours under an autogenous pressure of 5 bar.

The meta-phenoxybenzaldehyde was isolated from the reaction mixture as described in Example I, section (b) and obtained in a yield of 92%.

Comparative Experiment

A glass reactor was charged with a solution of hexamethylenetetramine (1 mol.) in water (417 ml) and then, at once and with stirring, with a mixture of meta-phenoxybenzyl chloride (0.32 mol.) and meta-phenoxybenzal chloride (0.35 mol.) as obtained in Example I, section (a). After stirring for three hours at 90°C, glacial acetic acid (417 ml) was added and the mixture kept under reflux (106°C) for 8 hours.

After cooling to 20°C the reaction mixture was extracted with two 150 ml portions of carbon tetrachloride, the combined extract phases were washed with water (80 ml) and the solvent was evaporated from the washed extract phase to give meta-phenoxybenzaldehyde in a yield of 90%.

Comparison with Example V shows that with the ethanol-water mixture a slightly higher yield of meta-phenoxybenzaldehyde is obtained after a shorter hydrolysis time.

EXAMPLE VI

A flask was charged with a solution of hexamethylenetetramine (0.30 mol.) in a 70%v/30%v propanol/water mixture (260 ml) and then, over a period of one hour at 60°C and with tirring, with a mixture of meta-phenoxybenzyl chloride (0.12 mol.) and meta-phenoxybenzal chloride (0.09 mol.) as obtained

0003066

in Example I, section (a). After stirring for a further hour at 60°C, water (16 ml) was added and the mixture kept under reflux (88°C) for 18 hours.

The meta-phenoxybenzaldehyde was isolated as described in Example I, section (b) and obtained in a yield of 73%.

1

## C L A I M S

1.    Process for the preparation of meta-aryloxybenzaldehydes, characterized in that in a first step a mixture of the corresponding meta-aryloxybenzyl halides and meta-aryloxybenzal halides is reacted with hexamethylenetetramine, and in a second step, the resulting product is hydrolyzed in the presence of a non-acidic solvent for the said product.

2.    Process according to claim 1, characterized in that the non-acidic solvent is an alkanol with up to four carbon atoms per molecule.

3.    Process according to claim 2, characterized in that the alkanol is ethanol.

4.    Process according to claim 2 or 3, characterized in that the hydrolysis is conducted in an alkanol-water mixture containing 40%v to 80%v of an alkanol with up to four carbon atoms per molecule.

5.    Process according to any one of the preceding claims, characterized in that the mixture of the meta-aryloxybenzyl halide and meta-aryloxybenzal halide used as starting material is prepared by a process which comprises halogenating the corresponding meta-aryloxytoluene with gaseous halogen at an elevated temperature in the presence of a free-radical initiator.

6.   Process according to claim 5, characterized in that a mixture of meta-aryloxybenzyl chloride and meta-aryloxybenzal chloride is prepared by a process which comprises contacting a meta-aryloxytoluene in a non-polar solvent at a temperature in the range of from 40 to 100°C with gaseous chlorine.

7.   Process according to claim 6, characterized in that the non-polar solvent is a halogenated hydrocarbon.

8.   Process according to claim 7, characterized in that the halogenated hydrocarbon is carbon tetrachloride.

9.   Process according to any one of claims 6, 7 or 8, characterized in that the free-radical initiator is azo-isobutyronitrile.

10.   Process according to any one of claims 6 to 9, characterized in that the reaction between the meta-aryloxytoluene and chlorine is stopped at a conversion in the range of from 95% to 99%, based on meta-aryloxytoluene.

11.   Process according to any one of the preceding claims, characterized in that the meta-aryloxybenzaldehyde is meta-phenoxybenzaldehyde.

12.   Meta-aryloxybenzaldehydes prepared by a process according to any one of the preceding claims.